# EUROPEAN PATENT SPECIFICATION

(11) **EP 0 567 613 B1**
(45) Date of publication and mention of the grant of the patent: **20.02.2002**
(21) Application number: 92922550.6
(22) Date of filing: 13.10.1992
(51) Int. Cl.: A61K 31/19, A61K 31/20, A61K 31/70, A61K 38/21, A61P 35/00

(54) **COMPOSITIONS FOR THERAPY AND PREVENTION OF CANCER**
ZUSAMMENSETZUNGEN ZUR BEHANDLUNG UND VORBEUGUNG VON KREBS
COMPOSITIONS DESTINEES AU TRAITEMENT ET A LA PREVENTION DU CANCER

(30) Priority: 21.10.1991 US 779744
(43) Date of publication of application: 03.11.1993
(60) Divisional application: 00126980.2
(73) Proprietor: The Government of the United States of America, as represented by the Secretary, Department of Health and Human Services, Rockville, MD 20852-3804 (US)
(72) Inventor: Samid, Dvorit, Rockville, MD 20850 (US)
(74) Representative: Eisenführ, Speiser & Partner
(86) International application number: US9208875
(87) International publication number: WO9307866

(56) References cited:
- EP-A- 0 069 232
- DE-A- 2 523 108
- EXPERIENTIA, vol.27, 1971 pages 860 - 861 W. NEISH 'Phenylacetic acid as a potential therapeutic Agent for the treatment of human cancer'
- DRUGS EXP. CLIN. RES., vol.12, 1986 pages 11 - 16 S.R. BURZYNSKI 'Preclinical studies on Antineoplaston AS2-1 and Antineoplaston AS2-5'
- MOL. CELL. ENDOCRINOL., vol.80, no.1-3, 1991 pages 183 - 192 Y. SHECHTER ET AL. 'Hydroxyphenyl acetate derivatives inhibit protein tyrosine kinase activity and proliferation in Nb2 rat lymphoma cells and insulin-induced lipogenesis in rat adipocytes'
- CANCER RES., vol.52, no.7, 1992 pages 1988 - 1992 D. SAMID ET AL. 'Phenylacetate: A novel nontoxic inducer of tumor cell differentiation'
- BR. J. HAEMATOL., vol.79, 10 October 1991 pages 81 - 83 D. SAMID ET AL. 'Interferon in combination with antitumourigenic phenyl derivatives: potentiation of IFN alpha activity in-vitro'
- N ENGL J MED (UNITED STATES), AUG 20 1992, VOL. 327, NO. 8, PAGE(S) 569-70, Dover GJ et al 'Increased fetal hemoglobin in patients receiving sodium 4- phenylbutyrate [letter]'
- BLOOD (UNITED STATES), SEP 15 1992, VOL. 80, NO. 6, PAGE(S) 1576-81, Samid D et al 'Induction of erythroid differentiation and fetal hemoglobin production in human leukemic cells treated with phenylacetate.'
- S. BUDAVARI ET AL. 'THE MERCK INDEX' 1989 , MERCK & CO., INC. , RAHWAY, N.J., USA * No. 4785: Hydroxyurea *
- J NATL CANCER INST (UNITED STATES), SEP 16 1992, VOL. 84, NO. 18, PAGE(S) 1398, Smigel K 'Non-toxic drug being tested to treat cancer and anemias [news]'
- BIOCHEM. BIOPHYS. RES. COMMUN., 1977, VOL. 77, PAGE(S) 1217-23 Ross, Philip D. et al 'Inhibition of sickle cell hemoglobin gelation by some aromatic compounds'
- BLOOD (UNITED STATES), JAN 1 1995, VOL. 85, NO. 1, PAGE(S) 43-9, Collins AF et al 'Oral sodium phenylbutyrate therapy in homozygous beta thalassemia: a clinical trial.'
- PHARMACOLOGIST, VOL. 20, NO. 3, 1978, P. 204 JONES G L 'ANTI SICKLING EFFECTS OF BETA DI ETHYLAMINOETHYLDIPHENYLPROPYL ACETATE SKF-525-A'
- NIGER. J. PHARM., 1981, VOL. 12, PAGE(S) 285-7 Erhun, Wilson O. et al 'Acetonyl esters of hydroxybenzoic acids as potential antisickling agents'
- CHEMICAL ABSTRACTS: Vol. No. 83, 1975, Abstract WO 53278s: MARCOT et al., entire reference.

## Description

This invention relates to the use of pharmaceutically acceptable derivatives of phenylacetic acid as medicament, particularly as antitumor agents.

Some of the most dreadful epidemics, inherited diseases and cancerous infections in the history of mankind are afflicting the world's people at a rapid and discomforting rate. These maladies are being caused in many instances by the increasing cases of cancer. When one pauses to reflect upon the devastating pain, suffering and ultimately death experienced by persons afflicted, these moments of reflection underscore the tremendous importance which must be accorded medical research. In response to the need to alleviate suffering and add comfort to human life, the scientific community throughout the world is searching for effective treatments to prevent or ameliorate diseases.

### DESCRIPTION OF RELATED DISCLOSURES

Phenylacetic acid (PAA) is a protein decomposition product found throughout the phylogenetic spectrum, ranging from bacteria to man. Highly conserved in evolution, PAA may play a fundamental role in growth control and differentiation. In plants, PAA serves as a growth hormone (auxin) promoting cell proliferation and enlargement at low doses (10⁻⁵-10⁻⁷ M), while inhibiting growth at higher concentrations. The effect on animal and human cells is less well characterized. In humans, PAA acid is known to conjugate glutamine with subsequent renal excretion of phenylacetylglutamine (PAG). The latter, leading to waste nitrogen excretion, has been the basis for using PAA or preferably its salt sodium phenylacetate (NaPA) in the treatment of hyperammonemia associated with inborn errors of ureagenesis. Clinical experience indicates that acute or long-term treatment with high NaPA doses is well tolerated, essentially free of adverse effects, and effective in removing excess glutamine. [Brusilow, S.W., Horwich, A.L. Urea cycle enzymes. Metabolic Basis of Inherited Diseases, Vol. 6:629-633 (1989)]. These characteristics should be of value in cancer intervention.

Glutamine is the major nitrogen source for nucleic acid and protein synthesis, and substrate for energy in rapidly dividing normal and tumor cells. Compared with normal tissues, most tumors, due to decreased synthesis of glutamine along with accelerated utilization and catabolism, operate at limiting levels of glutamine availability, and consequently are sensitive to further glutamine depletion. Considering the imbalance in glutamine metabolism in tumor cells and the ability of PAA to remove glutamine, PAA has been proposed as a potential antitumor agent, however, no data was provided to substantiate this proposal. [Neish, W.J.P. "Phenylacetic Acid as a Potential Therapeutic Agent for the Treatment of Human Cancer", Experentia, Vol. 27, pp. 860-861 (1971)].

Despite efforts to fight cancer, many malignant diseases that are of interest in this application still present a major challenge to clinical oncology. Prostate cancer, for example, is the second most common cause of cancer deaths in men. Current treatment protocols rely primarily on hormonal manipulations, however, in spite of initial high response rates, patients often develop hormone-refractory tumors, leading to rapid disease progression with poor prognosis. Overall, the results of cytotoxic chemotherapy have been disappointing, indicating a long felt need for new approaches to treatment of advanced prostatic cancer. Other diseases resulting from abnormal cell replication for example, metastatic melanomas, brain tumors of glial origin (e.g., astrocytomas), and lung adenocarcinoma, are all highly aggressive malignancies with poor prognosis. The incidence of melanoma and lung adenocarcinoma has been increasing significantly in recent years. Surgical treatment of brain tumors often fails to remove all tumor tissues, resulting in recurrences. Systemic chemotherapy is hindered by blood barriers. Therefore there is an urgent need for new approaches to the treatment of human malignancies such as advanced prostatic cancer, melanoma, brain tumors, and others.

The development of the methods of the present invention was guided by the hypothesis that metabolic traits that distinguish tumors from normal cells could potentially serve as targets for therapeutic intervention. Tumor cells show unique requirements for specific amino acids, of which glutamine would be the desired choice because of its major contribution to energy metabolism and to synthesis of purines, pyrimidines, and proteins. Along this line, promising antineoplastic activities have been demonstrated with glutamine-depleting enzymes such as glutaminase, and various glutamine antimetabolites, unfortunately, the clinical usefulness of these drugs has been limited by unacceptable toxicities. Consequently, the present invention focuses on PAA, a plasma component known to conjugate glutamine *in vivo*.

In addition to its effect on glutamine phenylacetate can induce tumor cells to undergo differentiation. (See examples 1-5, 8 and 9 herein). Differentiation therapy is a known desirable approach to cancer intervention. The underlying hypothesis is that neoplastic transformation results from defects in cellular differentiation. Inducing tumor cells to differentiate would prevent tumor progression and bring about reversal of malignancy. Several differentiation agents are known, but their clinical applications have been hindered by unacceptable toxicities and/or deleterious side effects.

Accordingly, a major object of the present invention is to provide compositions for treating various cancerous conditions with PAA and its pharmaceutically acceptable salts and derivatives.

Another object of the present invention is to provide compositions for the prevention of tumor progression and the development of malignant conditions in high risk individuals comprising prophylactically effective amounts of nontoxic agents such as phenylacetate and its pharmaceutically acceptable derivatives.

It is yet a further object to provide compositions for treating or preventing the onset of malignancies with a combination of phenylacetate (or its pharmaceutically acceptable derivatives) and various other therapeutic or preventive agents alone or in conjunction with conventional therapies.

### III. BRIEF SUMMARY OF THE INVENTION

Compositions of the present invention are defined in claims 1 and 16.

The present invention provides compositions for suppressing the growth of tumor cells in a host in need of such suppression comprising an amount of PAA or a pharmaceutically acceptable derivative thereof effective to suppress the growth of said tumor cells.

For the purpose of the present application, the PAA derivatives include its pharmacological acceptable salts, preferably sodium; analogs containing halogen substitutions, preferably chlorine or fluorine; analogs containing alkyl substitutions, preferably methyl or methoxy; precursors of phenylacetate, preferably phenylbutyrate; and natural analogs such as naphthylacetate.

The compounds of the present invention can be administered intravenously, enterally, parentally, intramuscularly, intranasally, subcutaneously, topically or orally. The dosage amounts are based on the effective inhibitory concentrations observed *in vitro* and *in vivo* in antitumorigenicity studies. The varied and efficacious utility of the compounds of the present invention is further illustrated by the finds that they may also be administered concomitantly or in combination with other antitumor agents such as hydroxyurea, 5-azacytidine, 5-aza-2'-deoxycytidine, suramin; retinoids; hormones; biological response modifiers, such as interferon and hematopoietic growth factors; and conventional chemo- and radiation therapy or various combinations thereof.

### IV. BRIEF DESCRIPTION OF THE DRAWINGS

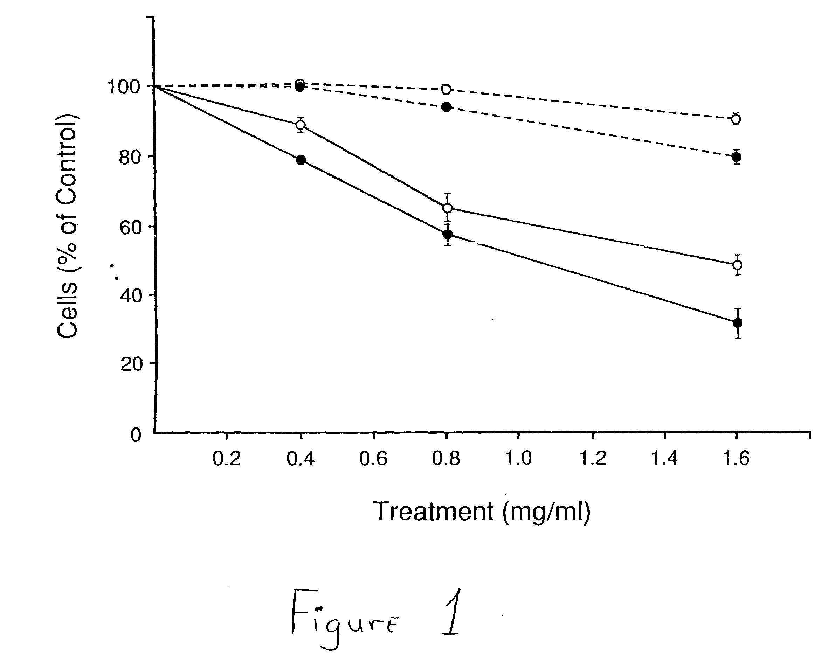

Figure 1 shows the inhibition of HL-60 leukemia and premalignant 10T1/2 cell proliferation by NaPA.

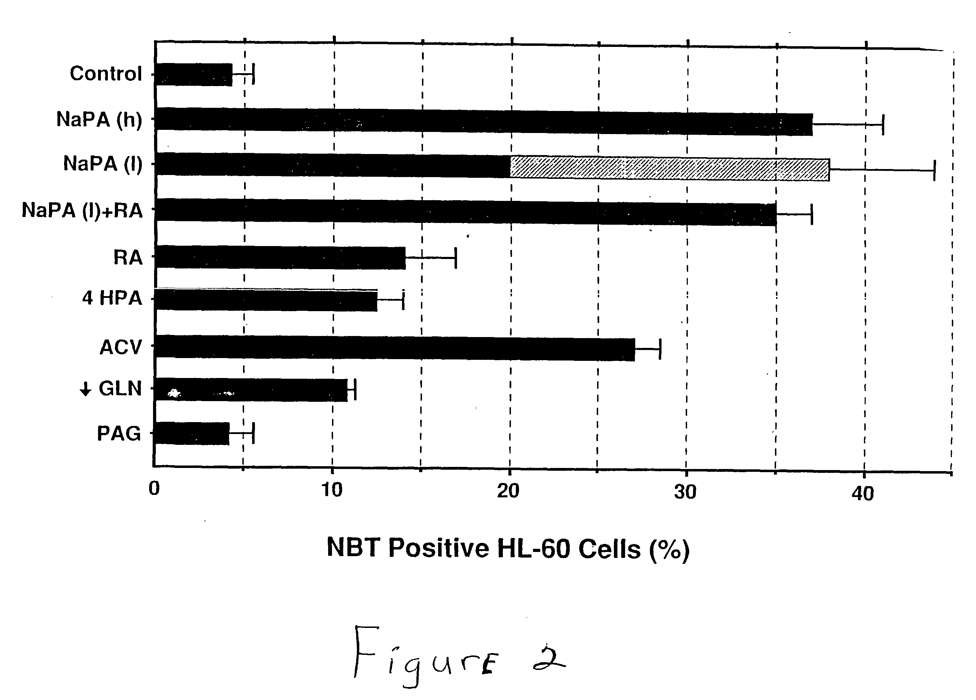

Figure 2 shows the induction of HL-60 cell differentiation. The number of NBT positive cells was determined after 4 [solid bars] or 7 days [hatched bar] of treatment. NaPA (h), 1.6 mg/ml; NaPA (1), 0.8 mg./ml. 4-hydroxy PA and PAG were used at 1.6 mg./ml. Potentiation by RA 10 nM was comparable to that by IFN gamma 300 IU/ml, and the effect of acivicin 3 µg/ml similar to DON 30 µg/ml. Glutamine Starvation (Gln, < 0.06 mM) was as described (18). Cell viability was over 95% in all cases, except for DON and acivicin (75% and 63%, respectively).

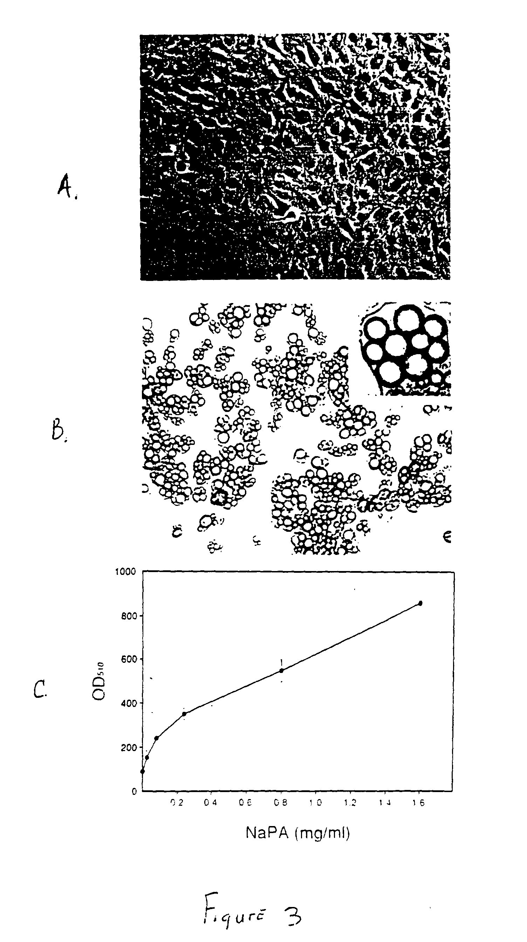

Figure 3 shows adipocyte conversion in 10T1/2 cultures. Confluent cultures were treated with NaPA for seven days. A. Untreated control [x100]; B. Cells treated with NaPA 0.8 mg/ml [x100]. The insert is showing a single cell with multipole lipid droplets [x400]. C. Quantitation of adipocytosis. Lipids stained with Oil-Red O were extracted with butanol, and the optical density at 510 nm determined. Increased lipid accumulation was evident with NaPA concentrations as low as 0.024 mg/ml.

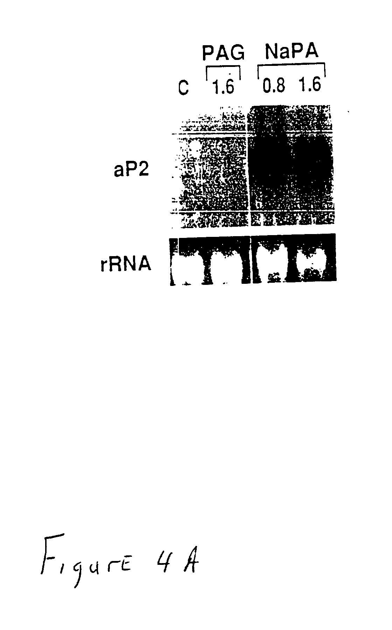

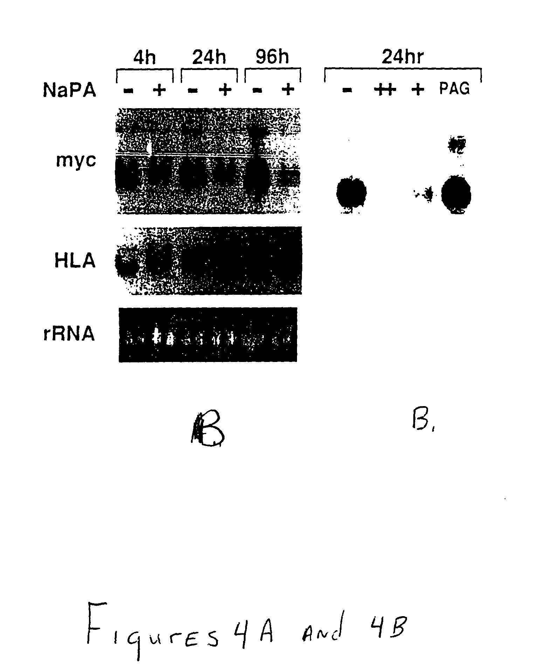

Figure 4 shows the modulation of gene by NaPA. Cytoplasmic RNA (20 µg/lane) was subjected to Northern blot analysis using ³²P-labeled specific DNA probes. Ethidium bromide-stained 285 rRNA indicates the relative amounts of total RNA in each lane. Figure A. Induction of a P2 expression in 10T1/2 cells. RNA from confluent cultures treated for 72 hrs with NaPA or PAG (mg/ml); C. untreated control. Figure B. Kinetics of myc inhibition and HLA-A induction in HL-60. Cells were treated with 0.8 mg/ml NaPA for the specified furcation (+); untreated controls (-). Figure C. Dose-dependent and specificity of effect. HL-60 cells treated with NaPA 1.6 mg/ml (++) and 0.8 mg/ml (+); PAG 1.6 mg/ml.

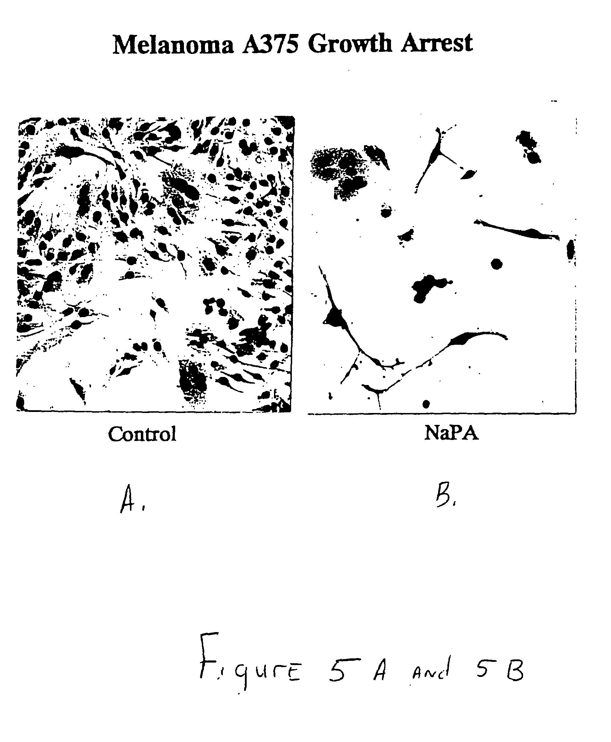

Figure 5 shows the inhibition of melanoma A375 cell growth and the induction of melanocyte differentiation with NaPA (5B) 5A shows the cell growth of the untreated cells.

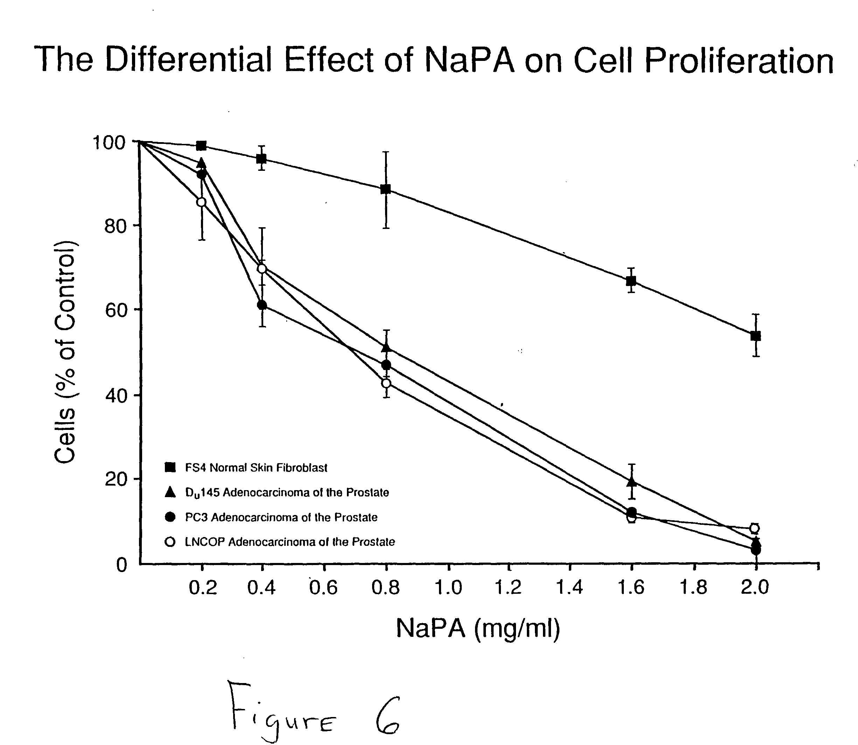

Figure 6 shows the effect of NaPA on cell proliferation. PC3; DU145; LNCap; and, FS4 cultures were treated with NaPA [ ] or PAG [ ] for four days.

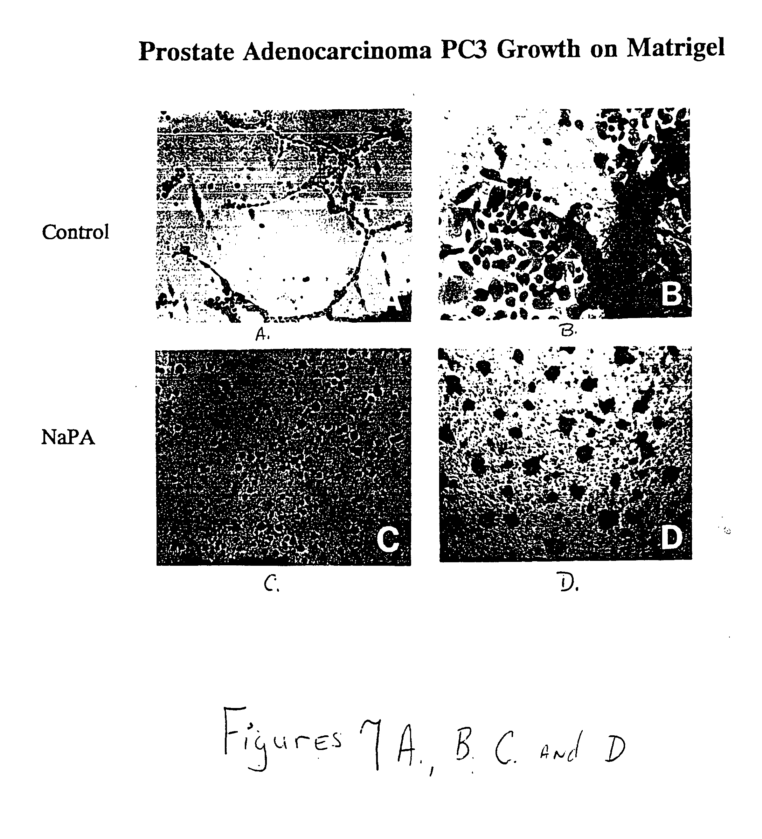

Figure 7 shows the growth of PC3 cells on matrigel. Four days after treatment with 800 µg/ml NaPA in T.C. plastic dishes, 5 x 10⁴ cells were replated onto 16 mm dishes (Costar) coated with 250 µl of matrigel. Pictures of controls, taken after 1 and 8 days (A and B, respectively), show the characteristic growth pattern of untreated cells, i.e., formation of net-like structures composed of actively replicating cells, which eventually degraded the matrigel and formed monolayers on the plastic surface beneath. In contract to controls, the NaPA-treated cells formed isolated small colonies which failed to degrade the matrigel barrier (picture C, taken 8 days after plating), resembling the behavior of normal human FS4 cells.

Figure 8 shows the inhibition of tumor cell invasion by NaPA cells treated in culture for 7 days were harvested and assayed for their invasive properties using a modified WYLW Chamber with a matrigel-coated filter. Results scored 6-24 hours later.

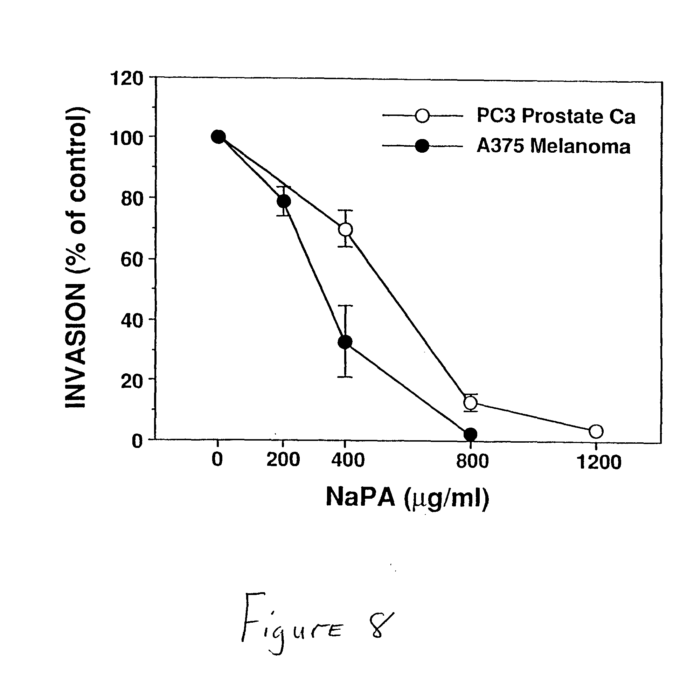

Figure 9 shows the prevention by NaPA of oncogene-induced neoplastic transformation. NIH 3T3 cells were transfected with cloned EJras DNA and treatment with NaPA started 24 hours later. Left: Untreated control cells at about 3 weeks after DNA transfer. Middle: control of cells with as oncogenes, showing the rate of spontaneous transformation. Right: Oncogene-transfected cells, heated with NaPA.

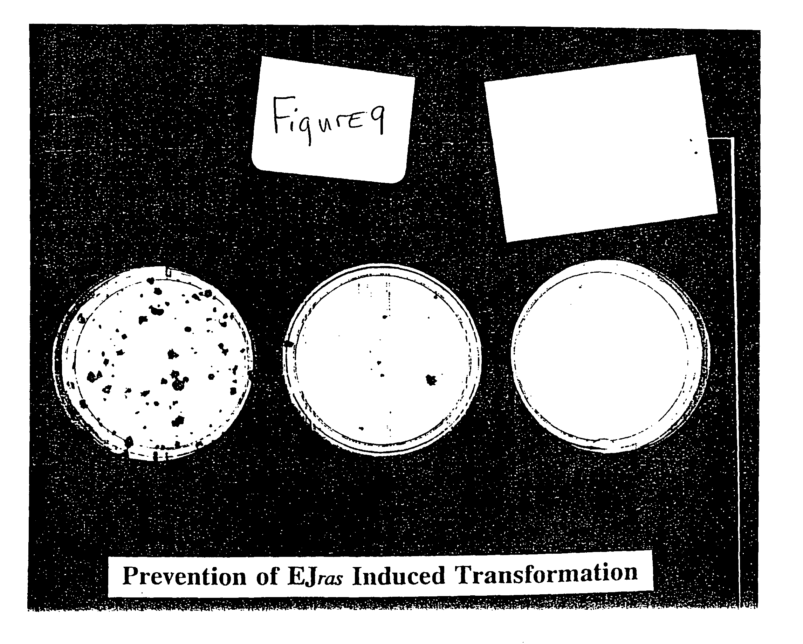

Figure 10 shows the prevention by NaPA of tumor progression induced by the chemotherapeutic drug 5-aza-2-deoxycytidine (5-AzadC) Premalignant 4C8 cells were treated in culture with 5-AzadC in the presence or absence of NaPA. One-two weeks later the cells were transplanted subcutaneously into athymic mice. Upper panel: mice injected the 5-AzadC-treated cells. Lower panel: mice injected with cells treated by both 5-AzadC and NaPA. Results recorded at about one month after cell transplantation.

### V. DETAILED DESCRIPTION OF THE INVENTION

According to the present invention, PAA, in particular its sodium salt NaPA has been found to be an excellent inhibitor of the growth of specific tumor cells, affecting the proliferation of the malignant cells while sparing normal tissues. Also, according to the present invention, NaPA has been found to induce tumor cell differentiation, thus offering a most desirable approach to cancer prevention and therapy. The exact mechanisms by which the compounds used in the method of this invention exert their effect is uncertain, one mechanism may involve depletion of plasma glutamine. Based on the data reported herein, it is believed that glutamine depletion alone cannot explain the molecular and phenotypic changes observed *in vitro* following exposure to NaPA. It will be understood, however, that the present invention is not to be limited by any theoretical basis for the observed results.

### VI. EXAMPLES

The herein offered examples, including experiments, provide methods for illustrating, without any implied limitation, the practice of this invention focusing on phenylacetate and its derivatives directed to A) cancer therapy and prevention.

### SECTION A.

### PHENYLACETATE IN CANCER PREVENTION AND MAINTENANCE THERAPY

Recent advances in molecular techniques allow the detection of genetic disorders associated with predisposition to cancer. Consequently, it is now possible to identify high-risk individuals as well as patients in remission with residual disease. Despite such remarkable capabilities, there is no acceptable prevention treatment. Chemopreventive drugs are needed also for adjuvant therapy, to minimize the carcinogenic effects of the prevailing anticancer agents and maintain tumor responses.

To qualify for use in chemoprevention, a drug should have antitumor activities, be nontoxic and well tolerated by humans, easy to administer (orally), and inexpensive. We suggest that NaPA may possess all of the above characteristics.

### 1. Prevention of Neoplastic Transformation - Oncogene Transfer Studies

NIH 3T3 cells carrying activated EJras oncogene (originally isolated from human bladder carcinoma) were used as a model to study the potential benefit of NaPA treatment to high risk individuals, in which predisposition is associated with oncogene activation. Cell treatment with NaPA was initiated 24-48 hr after oncogene transfer. Results, scored 14-21 days later, shows dose-dependent reduction in the formation of ras-transformed foci in cultures treated with NaPA. Molecular analyses indicated that the drug did not interfere with oncogene uptake and transcription, but rather prevented the process of neoplastic transformation. The effect was reversible upon cessation of treatment. In treated humans, however, the fate of the premalignant cells may be substantially different due to involvement of humoral and cellular immunity (see below).

### 2. Prevention of tumor progression by genotoxic chemotherapy

Current approaches to combat cancer rely primarily on the use of chemicals and radiation, which are themselves carcinogenic and may promote recurrences and the development of metastatic disease. One example is the chemotherapeutic drug 5-aza-2' deoxycytidine (5-AzadC). While this drug shows promise in treatment of some leukemias and severe inborn anemias, the clinical applications have been hindered by concerns regarding toxicity and carcinogenic effects. Our data indicate that NaPA can prevent tumor progression induced by 5-AzadC.

The experimental model involved nonmalignant 4C8a10 cells (revertants of Haras-transformed NIH3T3 fibroblasts). Transient treatment of the premalignant cells with 5-AzadC resulted in malignant conversion evident within 2 days, as determined by cell morphology, loss of contact inhibition and anchorage dependent growth in culture, acquired invasive properties and tumorigenicity in recipient athymic mice. Remarkably, NaPA prevented the development of the malignant phenotype in the 5-AzadC treated cultures.

**TABLE 1**

| | Tumor Formation^{a} | | Growth on matrigel^{b} |
|---|---|---|---|
| Treatment | Incidence | Size (mm) | |
| None | 3/8 | 1 (0.5-2) | - |
| 5-AzadC (0.1 µM) | 8/8 | 11.5 (4-19) | + |
| NaPA (1.5 mg/ml) | 0/8 | | - |
| 5-AzadC + NaPA (0.1 µM) (1.5mg/ml) | 0/8 | 0 | - |

| | | | |
|---|---|---|---|
| ^{a} Cells pretreated in culture were injected s.c. (5x10⁵ cells per site) into 3 month old female athymic nude mice (Division of Cancer Treatment, NCI Animal Program, | | | |

Frederick Cancer Research Facility). Results indicate the incidence (tumor bearing/injected animals), as well as tumor size as mean (range), determined after 3 weeks.

^{b} Cells were plated on top of matrigel (reconstituted basement membrane) and observed for malignant growth pattern, i.e., active replication, development of characteristic processes, and invasion.

### Anticipated Activity in Humans.

In terms of cancer prevention, the beneficial effect of NaPA on humans may be even more dramatic than that observed with the experimental models. In humans, NaPA is known to deplete circulating glutamine, an amino acid critical for the development and progression of cancer. The enzymatic reaction leading to glutamine depletion takes place in the liver and kidney; it is not clear whether or not glutamine depletion occurs in the cultured tumor cells. Moreover, molecular analysis revealed that NaPA can induce the expression of histocompatibility class I antigens, which are localized on the surface of tumor cells and affect the immune responses of the host. While the therapeutic benefit of NaPA observed in cultures is in some cases reversible upon cessation of treatment, in patients the tumor cells might eventually be eliminated by the immune system. Even if chemoprevention will require continuous treatment with NaPA, this would be acceptable considering the lack of toxicity.

Pharmaceutical compositions containing phenylacetate have been shown to cause reversal of malignancy and to induce differentiation of tumor cells. To demonstrate the capacity of drugs to induce differentiation of tumor cells, three *in vitro* differentiation model systems were used. (See sections A and D herein). The first system used a human promyelocytic leukemia cell line HL-60. This cell line represents uncommitted precursor cells that can be induced to terminally differentiate along the myeloid or monocytic lineage. In the second system, immortalized embryonic mesenchymal C3H 10T1/2 cells were used which have the capabilities of differentiating into myocytes, adipocytes, or chondrocytes. In the third system, human erythroleukemia cells (K562) were used which can be induced to produce hemoglobin.

### EXAMPLE 1

Referring now to the data obtained using the first system, the results of which are illustrated in Fig. 1, logarithmically growing HL-60 [-0-] and 10T1/2 [-0-] cells were treated for four days with NaPA[++] or phenylacetylglutamate (PAG) [---]. The adherent cells were detached with trypsin/EDTA and the cell number determined using a hemocytometer. Data points indicate the mean ± S.D. of duplicates from two independent experiments. The cell lines were obtained from the American Type Culture Collection and maintained in RPMI 1640 (HL-60) or Dulbecco's Modified Eagle's Medium (10T1/2) supplemented with 10% heat inactivated fetal calf serum (Gibco Laboratories), 2 mM L-Glutamine, and antibiotics. PAA (Sigma, St. Louis MO) and PAG were each dissolved in distilled water, brought to pH 7.0 by the addition of NaOH, and stored in -20°C until used. As demonstrated in Fig. 1, NaPA treatment of the HL-60 and 10T1/2 cultures was associated with dose dependent inhibition of cell proliferation.

### EXAMPLE 2

To further evaluate the effectiveness of NaPA as an inducer of tumor cell differentiation, the ability of NaPA to induce granulocyte differentiation in HL-60 was investigated. The ability of cells to reduce nitroblue tetrazolium (NBT) is indicative of oxidase activity which is characteristic of the more mature forms of human bone marrow granulocytes. NBT reduction thus serves as an indicator of granulocyte differentiation. In Fig. 2, the number of NBT positive cells was determined after 4 days [solid bars] or 7 days [hatched bards] of treatment. NaPA (h), 1.6 mg/ml; NaPA (1), 0.8 mg/ml. 4-hydroxyphenylacetate (4HPA) and PAG were used at 1.6 mg/ml. Potentiation by retinoic acid (RA) 10 nM was comparable to that by interferon gamma 300 IU/ml. The direction of differentiation towards granulocytes in cultures treated with NaPA, whether used alone or in combination with RA, was confirmed by microscopic evaluation of cells stained with Wright Stain and the lack of nonspecific esterase activity. The effect of acivicin (ACV) 1 µg/ml was similar to 6-diazo-5-oxo-L-norleucine (DON) 25 µg/ml. Glutamine starvation (Gln, < 0.06 mM) was as described. Cell viability determined by trypan blue exclusion was over 95% in all cases, except for DON and ACV which were 75% and 63%, respectively. DON, ACV and HPA are glutamine antagonists. As illustrated in Fig. 2, it is clear that NaPA is capable of inducing granulocyte differentiation in HL-60. As further illustrated in Fig. 2, differentiation of HL-60, assessed morphologically and functionally, was sequential and could be further enhanced by the addition of low doses of retinoic acid [RA, 10 nM) or interferon gamma (300 IU/ml). After seven days of NaPA treatment, or four days, when combined with RA, the HL-60 cultures were composed of early stage myelocytes and metamyelocytes (30-50%), as well as banded and segmented neutrophils (30-40%) capable of NBT.

Pharmacokinetic studies in children with urea cycle disorders indicate that infusion of NaPA 300-500 mg/kg/day, a well tolerated treatment, results in plasma levels of approximately 800 µg/ml. Brusilow, S.W. et al. Treatment of episodic hyperammonemia in children with inborn errors of urea synthesis. The New England Journal of Medicine. 310:1630-1634 (1984). This same concentration was shown to effectively induce tumor cell differentiation in the present experimental system.

### EXAMPLE 3

That NaPA is capable of inducing adipocyte conversion in 10T1/2 cultures is illustrated in Fig. 3. Confluent cultures were treated with NaPA for seven days. Upper: Untreated control [x100]; Middle: Cells treated with NaPA 0.8 mg/ml. The insert shows a single cell with multiple lipid droplets [x400]. Lower: Quantitation of adipocytosis. Cells were fixed with 37% formaldehyde and stained with Oil-Red O. The stained intracellular lipid was extracted with butanol, and the optical density was determined using a Titertek Multiskan MC, manufactured by Flow Laboratories, at a wavelength 510 nm. Increased lipid accumulation was evident in cells treated with as little as 0.024 mg/ml of NaPA. The results in Fig. 3 show that differentiation was dose and time-dependent, and apparently irreversible upon cessation of treatment. NaPA at 800 µg/ml was quite efficient and totally free of cytotoxic effect. In the 10T1/2 model, adipocyte conversion involved over 80% of the cell population. It was noted that higher drug concentrations further increased the efficiency of differentiation as well as the size of lipid droplets in each cell.

It is known that glutamine conjugation by NaPA is limited to humans and higher primates and that in rodents NaPA binds glycine. [James, M.O. et al. The conjugation of phenylacetic acid in man, sub-human primates and some nonprimate species. Proc. R. Soc. Lond. B. 182:25-35 (1972).] Consequently, the effect of NaPA on the mouse 10T1/2 cell line could not be explained by an effect on glutamine. In agreement, neither glutamine starvation nor by treatment with glutamine antagonists such as DON and ACV resulted in adipocyte conversion.

### EXAMPLE 4

**Table 2**

| **Phenylacetate and Derivatives: Induction of cellular differentiation in premalignant 10T1/2 cells** | | |
|---|---|---|
| **Compounds** (sodium salts) | **Differentiation at 1 mM** (%) | **DC50*** (mM) |
| Phenylacetate | 65 | 0.7 |
| 1-naphthylacetate | >95 | <0.1 |
| 3-chlorophenylacetate | 80 | 0.5 |
| 4-chlorophenylacetate | 50 | 1.0 |
| 2,6-dichlorophenylacetate | 75 | 0.5 |
| 4-fluorophenylaceatae | 65 | 0.7 |

| | | |
|---|---|---|
| * DC50, concentration of compound causing 50% differentiation | | |

### Potential clinical use of phenylacetate and derivatives

As shown in the table, phenylacetate and its derivatives efficiently induced lipid accumulation and adipocyte (fat cell) differentiation in premalignant cells. This and other results indicate that the tested compounds might be of value in:
1. Cancer prevention. Non-replicating, differentiated tumor cells are not likely to progress to malignancy.
2. Differentiation therapy of malignant and pathological nonmalignant conditions.
3. Treatment of lipid disorders, in which patients would benefit from increased lipid accumulation.

It is known that studies in plants reveal that NaPA can interact with intracellular regulatory proteins and modulate cellular RNA levels. In an attempt to explore the possible mechanism of action, Northern blot analysis of HL-60 and 10T1/2 cells was performed according to conventional methods. As shown in Fig. 4a, cytoplasmic RNA was extracted, separated and analyzed (20 ≤ g/lane) from confluent cultures treated for 72 hrs with NaPA or PAG (mg/ml); C is the untreated control. The aP2 cDNA probe was labeled with [³²P]dCTP (NEN) using a commercially available random primed DNA labeling kit. Ethidium bromide-stained 28S rRNA indicates the relative amounts of total RNA in each lane.

The results of the Northern blot analysis of HL-60 and 10T1/2 cells, illustrated in Fig. 4a, showed marked changes in gene expression shortly after NaPA treatment. Expression of the adipocyte-specific aP2 gene was induced within 24 hrs in treated 10T1/2 confluent cultures reaching maximal mRNA levels by 72 hrs.

### EXAMPLE 5

In HL-60, cell transformation has been linked to myc amplification and overexpression, and differentiation would typically require down regulation of myc expression. [Collins, S.J. The HL-60 promyelocytic leukemia cell line: Proliferation, differentiation, and cellular oncogene expression. Blood. 70:1233-1244 (1987)]. To demonstrate the kinetics of myc inhibition and HLA-A induction, Northern blot analysis of cytoplasmic RNA (20 µg/lane) was carried out on cells treated with NaPA and PAG for specified durations of time and untreated controls (-). The dosedependency and specificity of the effect of NaPA is also illustrated in Fig. 4b. Two concentrations of NaPA, 1.6 mg/ml (++) and 0.8 mg/ml (+), and PAG at 1.6 mg/ml was investigated. The 32P-labeled probes used were myc 3rd exon (Oncor) and HLA-A3 Hind III/EcoRI fragment. As the data show in Fig. 4c, NaPA caused a rapid decline in the amounts of myc mRNA. This occurred within 4 hours of treatment, preceding the phenotypic changes detectable by 48 hrs, approximately two cell cycles, after treatment. Similar kinetics of myc inhibition have been reported for other differentiation agents such as dimethyl sulfoxide, sodium butyrate, bromodeoxyuridine, retinoids, and 1,25-dihydroxyvitamin D3. The results observed in Figs. 4b & 4c suggest that down regulation of oncogene expression by NaPA may be responsible in part for the growth arrest and induction of terminal differentiation. In addition, it is evident in Fig. 4b that NaPA treatment of the leukemic cells was associated with time- and dose-dependent accumulation of HLA-A mRNA coding for class I major histocompatibility antigens. It is believed that this may enhance the immunogenicity of tumors *in vivo.*

### EXAMPLE 6

Further support for the use of NaPA as a non-toxic inducer of tumor cell differentiation was found in the ability of NaPA to promote hemoglobin biosynthesis in erythroleukemia cells. It is known that K562 leukemic cells have a nonfunctional beta globin gene and, therefore, do not normally make much hemoglobin. When K562 human erythroleukemia cells were grown in the presence of NaPA at 0.8 and 1.6 mg/ml concentrations, hemoglobin accumulation, a marker of differentiation, was found to increase 4-9 fold over the control cells grown in the absence of NaPA. Hemoglobin accumulation was determined by Benzidine staining of cells for hemoglobin and direct quantitation of the protein. The results of this study are reported in Table 3.

It has been shown that high concentrations of NaPA inhibit DNA methylation in plants. [Vanjusin, B.J. et al. Biochemia 1,46:47-53 (1981)]. Alterations in DNA methylation can promote oncogenesis in the evolution of cells with metastatic capabilities. [Rimoldi, D. et al. Cancer Research. 51:1-7 (1991)]. These observations raised some concerns regarding potential long-term adverse effects with the use of NaPA. To determine the potential tumorigenicity of NaPA, a comparative analysis was performed using NaPA and a known hypomethylating agent 5-aza-2'-deoxycytidine (5-AzadC).

Premalignant cells (3-4 x 10⁵) were plated in 75 cm² dishes and 5-AzadC 0.1 µM was added to the growth medium at 20 and 48 hrs after plating. The cells were then subcultured in the absence of the nucleoside analog for an additional seven weeks. Cells treated with NaPA at 1.6 mg/ml were subcultured in the continuous presence of the drug. For the tumorigenicity assay, 4-5 week-old female athymic nude mice were inoculated s.c. with 1 x 10⁶ cells and observed for tumor growth at the site of injection.

The results set forth in Table 1 shown that NaPA, unlike the cytosine analog, did not cause tumor progression.

**Table 3.**

| Tumorigenicity of C3H 10T1/2 Cells in Athymic Mice | | | |
|---|---|---|---|
| Treatment Time (weeks) | Tumors | Incidence (positive/ injected mice) | Diameter (mm ± S.D.) |
| None 13 | | 0.8 | 0 |
| | | | |
| 5-AzadC 8 | | 8/8 | 5.5 ± 2.5 |
| | | | |
| NaPA 13 | | 0/8 | 0 |

The transient treatment of actively growing 10T1/2 cells with 5-AzadC resulted in the development of foci of neoplastically transformed cells with a frequency of about 7x10⁻⁴. These foci eventually became capable of tumor formation in athymic mice. By contrast, actively replicating 10T1/2 cultures treated for seven weeks with NaPA, 800-1600 µg/ml, differentiated solely into adipocytes, forming neither neoplastic foci *in vitro* nor tumors in recipient mice.

Furthermore, experiments have demonstrated that NaPA can prevent spontaneous or 5-AzadC-induced neoplastic transformation, thus suggesting a potential role in cancer prevention. It is known that the treatment of premalignant 4C8 and 10T1/2 cells with carcinogens such as 5-AzadC produces malignant conversion of the respective cells. When 4C8 [Remold: et al., Cancer Research, 51:1-7 (1990)] and 10T1/2 cells were exposed to 5-AzadC, malignant conversion was evident in two days and two weeks, respectively. NaPA (0.8-1.6 mg/ml) prevented the appearance of the malignant phenotype, as determined by cell morphology, contact inhibition and anchorage dependent growth in culture.

### EXAMPLE 7

The K562 erythroleukemia line serves as a model for inherited anemias that are associated with a genetic defect in the beta globin gene leading to severe β-chain hemoglobinopathies.

The results reported in Table 3 also show that there is a synergistic affect when leukemia cells are exposed to NaPA in combination with interferon alpha, a known biological response modifier or with the chemotherapeutic drug hydroxyurea (HU).

**Table 4.**

| Induction of Hemoglobin Synthesis in Erythroleukemia K562 cells | | |
|---|---|---|
| Treatment | Benzidine Positive Cells* (%) | Cell Viability (%) |
| Control | 1.8 | > 95 |
| | | |
| NaPA 0.8 mg/ml | 6.0 | |
| 1.6 mg/ml | 17.1 | |
| | | |
| Interferon 500 IU/ml | 13.5 | |
| | | |
| HU 100 µM | 17.2 | |
| | | |
| NaPA (0.8 mg/ml) + HU or IFN | 40-42 | |

| | | |
|---|---|---|
| * Results at seven days of treatment. | | |

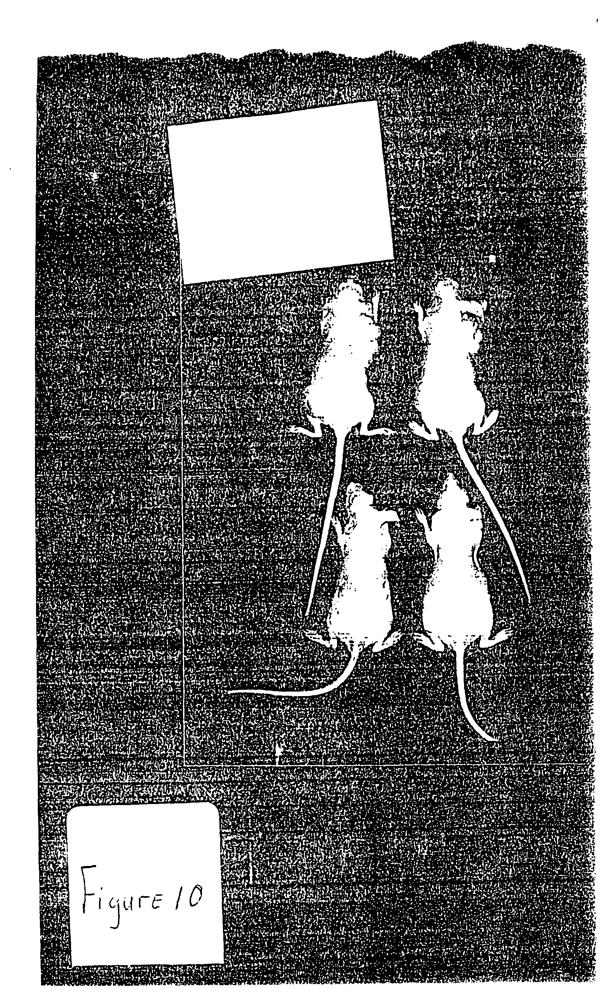

Analysis of gene transcripts showed accumulation of mRNA coding for gamma globin, (Figure 11) the fetal form of globin. This was confirmed at the protein level (Figure 12).

Using the erythroleukemia K562 cell line described above it was found that 4 hydroxyphenylacetate was as effective as NaPA in inducing fetal hemoglobin accumulation, but was less inhibitory to cell proliferation. In contrast, some other analogs such as 2,4- or 3,5-dihydroxyphenylacetate were found to be highly toxic (Please see section III, herein for further discussion).

### EXAMPLE 8

The effectiveness of NaPA as an antitumor agent was further evaluated in a variety of experimental models. Studies of depth were performed with two androgenindependent human prostate adenocarcinoma cell lines, PC3 and DU145, established from bone and brain metastases, respectively. NaPA treatment of the prostatic cells resulted in concentration-dependent growth arrest, accompanied by cellular swelling and accumulation of lipid. The results of this study are shown in Fig. 7. As illustrated therein, an IC₅₀ for NaPA occurred at 600-800 µg/ml. Significantly higher doses were needed to affect the growth of actively replicating normal human FS4 skin fibroblasts, indicating a selective cytostatic effect of the drug.

### EXAMPLE 9

It is known that PC3 cells are invasive *in vitro* and metastatic in recipient athymic mice. [Albini, A. et al. A rapid *in vitro* assay for quantitating the invasive potential of tumor cells. Cancer Res. 47:3239-3245 (1987)]. The invasiveness of PC3 cells which is indicative of their malignant phenotype can be assessed by their ability to degrade and cross tissue barriers such as matrigel, a reconstituted basement membrane. Untreated PC3 cells and PC3 cells treated with NaPA for 4 days in culture were quantitatively analyzed in a modified Boyden chamber containing a matrigel-coated filter with FS4 conditioned medium as a chemoattractant. After 4 days of treatment with 800 µg/ml of NaPA in T.C. plastic dishes, 5 x 10⁴ cells were replated onto 16 mm Costar dishes coated with 250 ≤ 1 of matrigel. In Fig. 7, pictures of controls taken after 1 and 8 days, A and B, respectively, shown the characteristic growth pattern of untreated cells, i.e., formation of net-like structures composed of actively replicating cells which eventually degraded the matrigel and formed monolayers on the plastic surface beneath. In contrast to the controls, the NaPA treated cells formed isolated small colonies which resembled normal human FS4 cells as shown in C and D taken 8 days after plating. The NaPA treated cells failed to degrade the matrigel barrier. The formation of small noninvasive colonies on top of the matrigel is indicative of loss of malignant properties following treatment. Results of the *in vitro* invasion assays correlate highly with the biological behavior of cells *in vivo.*

### EXAMPLE 10

Indeed, PC3 cells treated with NaPA for one week in culture, in contrast to untreated cells or those treated with PAG, failed to form tumors when transplanted s.c. into athymic mice. These results are shown in Table 5.

**Table 5.**

| Tumorigenicity of Prostatic PC3 Cells in Nude Mice | | | |
|---|---|---|---|
| TREATMENT (mg/ml) | Incidence | Diameter (mm ± S.D.) | Weight (mg) |
| | | | |
| None | 7/7 | 9 ± 3 | 285 ± 60 |
| | | | |
| NaPA 0.8 | 1/7 | 2 | 50 |
| | | | |
| PAG 0.8 | 3/4 | 8 ± 2 | 245 ± 35 |

PC3 cells were pretreated for 1 week in culture and then injected (2 x 10⁵ cells/animal) s.c. into 4-5 week-old female athymic nude mice. The results in Table 5 indicate the incidence of tumor bearing animals/injected animals as well as tumor size measured as mean diameter ± S.D. 8 weeks later. The data in Table 4 are a summary of two independent experiments.

### EXAMPLE 11

To further substantiate the phenotypic changes observed in the NaPA treated prostatic PC3 cells, Northern blot analysis revealed that NaPA inhibited the expression of collagenase type IV, one of the major metalloproteases implicated in degradation of basement membrane components, tumor cell invasion, and metastasis. Furthermore, it was found that NaPA treated prostatic PC3 cells showed an increase in the level of HLA-A mRNA which codes for major histocompatibility class I antigen known to affect tumor immunogenicity *in vivo.*

NaPA in Combination with Suramin

**Table 6**

| Malignant Melanoma A375 | | |
|---|---|---|
| Treatment (µg/ml) | Growth (% of control) | Viability (%) |
| | | |
| None | 100 | > 95 |
| | | |
| NaPA 400 | 63.3 | > 95 |
| | | |
| Suramin 38 | 78.3 | > 95 |
| 75 | 56.8 | > 95 |
| 150 | 38.6 | 92 |
| 300 | 26.6 | 82 |
| | | |
| NaPA (400) + Suramin (38) | 45.5 | > 95 |
| + Suramin (75) | 30.1 | 94 |
| + Suramin (150) | 21.8 | 92 |

**Table 7**

| Prostate Adenocarcinoma PC3 | | |
|---|---|---|
| Treatment (µg/ml) | Growth (% of control) (%) | Viability |
| | | |
| None | 100 | >95 |
| | | |
| NaPA 800 | 59.6 | >95 |
| | | |
| Suramin 75 | 58.5 | no data |
| 150 | 46.5 | no data |
| 300 | 31.0 | no data |
| | | |
| NaPA (800) + Suramin (75) | 24.2 | 90 |
| + Suramin (150) | 10.9 | 64 |

NaPA was found to significantly potentiate the therapeutic effect of suramin, the only experimental drug known to be active against prostate cancer.

It is known that a disease state characterized by the presence of benign hyperplastic lesions of the prostate exists as a separate disease entity and has been identified in many patients that progress to a diagnosis of prostatic cancer. Based on the above, it is anticipated that NaPA, in addition to being effective in the treatment of prostatic cancer, would be effective in treating patients having benign hyperplastic prostatic lesions.

Further experiments demonstrated that NaPA appears to have broad antitumor activity affecting a wide spectrum of malignancies. The experimental data indicate presented in Table 5 that NaPA 0.4-0.8 mg/ml caused about 50% inhibition of growth in treated adenocarcinoma of the prostate cell lines PC3 and DU145, melanoma A375 and SK MEL 28, lung adenocarcinoma H596 and H661, and astrocytoma U87, U373, and 343. Somewhat higher concentrations (1.0-1.5 mg/ml) were needed to cause a similar inhibition of squamous cell carcinoma A431, breast tumor MCS-7, osteosarcoma KRIB, and fibrosarcoma V7T. Typically, NaPA treatment was associated with growth arrest, induction of differentiation markers, reduced invasiveness *in vitro,* and loss of tumorigenicity in nude mice.

**Table 8**

| RESPONSES OF DIFFERENT TUMOR CELL LINE TO NaPA TREATMENT | | | |
|---|---|---|---|
| # | Tumor Cell Line | | % Inhibition by NaPA 0.8 mg/ml^{a} |
| | | | |
| 1 | Melanoma | A375 | ≥ 70 |
| | | SK MEL 28 | > 50 |
| | | | |
| 2 | Prostatic Ca^{b} | PC3 | ≥ 50 |
| | | DU145 | ≥ 50 |
| | | LaNCoP | > 50 |
| | | | |
| 3 | Astrocytoma | U87 | ≥ 50 |
| | | U343 | ≥ 50 |
| | | U373 | ≥ 50 |
| | | | |
| 4 | Kaposi's Sarcoma | KS | ≤ 40 |
| | | | |
| 5 | Leukemia | HL-60 | ≤ 40 |
| | | | |
| 6. | Leukemia | K562 | ≤ 30 |
| | | | |
| 7 | Breast Cancer | MCF-7 | ≤ 30 |
| | | | |
| 8 | Osteosarcoma | KRIB | ≤ 30 |
| | | HOS | < 20 |
| | | | |
| 9 | Fibrosarcoma | V7T | ≤ 30 |
| | | RS485 | ≤ 30 |
| | | | |
| 10 | Squamous Cancer of Head & Neck | A431 | < 30 |

| | | | |
|---|---|---|---|
| ^{a} Pharmacologically attainable concentration | | | |
| ^{b} Carcinoma | | | |

### Of major interest in Table 8 are the following:

# 1-3 Tumor cells show significant response i.e., ≥ 50 inhibition of proliferation within one week of treatment.
# 4 KS, an HIV-associated disorder, may be more dramatically affected by NaPA in humans, due to inhibition of HIV expression and of essential growth factors released by infected lymphocytes.
# 5,6 The treated HL-60 promyelocytic leukemic cells undergo terminal differentiation, a desirable outcome of chemotherapy. In the K562 erythroleukemia, NaPA induced reversible erythroid differentiation with no significant growth arrest (<30); thus the K562 data is of interest with respect to treatment of certain anemias, not cancer.

### Less attractive:

# 7-10 For effective responses, the tumors may require much higher drug concentrations if used alone.

Although some of the malignant cell lines seem more sensitive than others, all were significantly more affected by NaPA when compared to normal or benign cells. For example, NaPA inhibited the growth of malignant osteosarcoma (KRIB) cells more so than benign osteosarcoma-derived HOS cells. A differential effect was seen also in ras-transformed fibrosarcoma V7T, when compared to the parental non-tumorigenic N1H3T3 cells. As to normal human cells, as much as 2-4 mg/ml of NaPA were needed to cause a significant inhibition of growth to primary human skin FS4 fibroblasts. It should be noted that the treatment was not toxic to either the malignant or the normal cells.

The concentration range found to selectively suppress malignant growth can be readily obtained in the clinical setting without causing significant side effects. Intravenous infusion of humans with NaPA at 250-500 mg/kg/day which results in plasma levels of 600-800 µg/ml has been found to be a well tolerated treatment. Cytotoxicity in tissue culture was observed when the NaPA concentration was > 3 mg/ml.

### Contemplated Models of Drug Administration

NaPA may be administered locally or systemically. Systemic administration means any mode or route of administration which results in effective levels of active ingredient appearing in the blood or at a site remote from the site of administration of said active ingredient.

The pharmaceutical formulation for systemic administration according to the invention may be formulated for intravenous, intramuscular, sub-cutaneous, oral, nasal, enteral, parenteral or topical administration. In some cases, combination of types of formulations may be used simultaneously to achieve systemic administration of the active ingredient.

Suitable formulations for oral administration include hard or soft gelatin capsules, dragees, pills, tablets, including coated tablets, elixirs, suspensions, and syrups or inhalations.

Solid dosage forms in addition to those formulated for oral administration include rectal suppositories.

The compounds of the present invention may also be administered in the form of an implant.

Suitable formulations for topical administration include creams, gels, jellies, mucilages, pastes and ointments.

Suitable injectable solutions include intravenous, subcutaneous, and intramuscular injectable solutions. The compounds of the present invention may also be administered in the form of an infusion solution or as a nasal inhalation or spray.

The compounds of the present invention may also be used concomitantly or in combination with selected biological response modifiers, e.g., interferons, interleukins, tumor necrosis factor, glutamine antagonists, hormones, vitamins, as well as anti-tumor agents and hematopoietic growth factors, discussed above.

It has been observed that NaPA is somewhat malodorous. Therefore, it may be preferable to administer this compound in the presence of any of the pharmaceutically acceptable odor-masking excipients or as its precursor phenylbutyrate which has no offensive odor.

The PAA and its pharmaceutically acceptable derivatives to be used as antitumor agents can be prepared easily using pharmaceutical materials which themselves are available in the art and can be prepared by established procedures. The following preparations are illustrative of the preparation of the dosage forms of the present invention, and are not to be construed as a limitation thereof.

### EXAMPLE 12

### PARENTERAL SOLUTION

A sterile aqueous solution for parenteral administration containing 200 mg/ml of NaPA for treating a neoplastic disease is prepared by dissolving 200 g. of sterilized, micronized NaPA in sterilized Normal Saline Solution, qs to 1000 ml. The resulting sterile solution is placed into sterile vials and sealed. The above solution can be used to treat malignant conditions at a dosage range of from about 100 mg/kg/day to about 1000 mg/kg/day. Infusion can be continuous over a 24 hour period.

### EXAMPLE 13

### PARENTERAL SOLUTION

A sterile aqueous solution for parenteral administration containing 50 mg/ml of NaPA is prepared as follows:

| Ingredients | Amount |
|---|---|
| NaPA, micronized | 50 g. |
| | |
| | |
| Benzyl alcohol | 0.90% w/v |
| Sodium chloride | 0.260% w/v |
| Water for injection, qs | 1000 ml |

The above ingredients, except NaPA, are dissolved in water and sterilized. Sterilized NaPA is then added to the sterile solution and the resulting solution is placed into sterile vials and sealed. The above solution can be used to treat a malignant condition by administering the above solution intravenously at a flow rate to fall within the dosage range set forth in Example 12.

### EXAMPLE 14

### PARENTERAL SOLUTION

A sterile aqueous solution for parenteral administration containing 500 mg/ml of sodium phenylbutyrate is prepared as follows:

| Ingredients | Amount |
|---|---|
| Sodium phenylbutyrate | 500 g. |
| Dextrose | 0.45% w/v |
| Phenylmercuric nitrate | 0.002% w/v |
| Water for injection, qs | 1000 ml. |

The preparation of the above solution is similar to that described in Examples 12 and 13.

### EXAMPLE 15

### TABLET FORMULATION

A tablet for oral administration containing 300 mg of NaPA is prepared as follows:

| Ingredients | Amount |
|---|---|
| NaPA | 3000 g. |
| Polyvinylpyrrolidone | 225 g. |
| Lactose | 617.5 g. |
| Stearic acid | 90 g. |
| Talc | 135 g. |
| Corn starch | 432.5 g. |
| Alcohol | 45 L |

NaPA, polyvinylpyrrolidone and lactose are blended together and passed through a 40-mesh screen. The alcohol is added slowly and the granulation is kneaded well. The wet mass is screened through a 4-mesh screen, dried overnight at 50°C and screened through a 20-mesh screen. The stearic acid, talc and corn starch is bolted through 60-mesh screen prior to mixing by tubing with the granulation. The resulting granulation is compressed into tablets using a standard 7/16 inch concave punch.

### EXAMPLE 16

### TABLET FORMULATION

A tablet for oral administration containing 200 mg of sodium phenylbutyrate is prepared as follows:

| Ingredients | Amount |
|---|---|
| Sodium phenylbutyrate | 2240 g. |
| Compressible sugar (Di-Pac) | 934 g. |
| Sterotex | 78 g. |
| Silica gel (Syloid) | 28 g. |

The above ingredients are blended in a twin-shell blender for 15 minutes and compressed on a 13/22 inch concave punch.

### EXAMPLE 17

### INTRANASAL SUSPENSION

A 500 ml sterile aqueous suspension is prepared for intranasal installation as follows:

| Ingredients | Amount |
|---|---|
| NaPA, micronized | 30.0 g. |
| Polysorbate 80 | 2.5 g. |
| Methylparaben | 1.25 g. |
| Propylparaben | 0.09 g. |
| Deionized water, qs | 500 ml |

The above ingredients, with the exception of NaPA, are dissolved in water and sterilized by filtration. Sterilized NaPA is added to the sterile solution and the final suspensions are aseptically filled into sterile containers.

### EXAMPLE 18

### OINTMENT

An ointment is prepared from the following ingredients:

| Ingredients | Amount |
|---|---|
| NaPA | 10 g. |
| Stearyl alcohol | 4 g. |
| White wax | 8 g. |
| White petrolatum | 78 g. |

The stearyl alcohol, white wax and white petrolatum are melted over a steam bath and allowed to cool. The NaPA is added slowly to the ointment base with stirring.

### EXAMPLE 19

### LOTION

| Ingredient | Amount |
|---|---|
| Sodium phenylbutyrate | 1.00 g. |
| Stearyl methylcellulose (4,500) solution (2%) | 25.00 ml |
| Benzalkonium chloride | 0.03 g. |
| Sterile water | 250.00 ml |

The benzalkonium chloride is dissolved in about 10 ml. of sterile water. The sodium phenylbutyrate is dispersed into methylcellulose solution by means of vigorous stirring. The methylcellulose (4,500) used is a high viscosity grade. The solution of benzalkonium chloride is then added slowly while stirring is continued. The lotion is then brought up to the desired volume with the remaining water. Preparation of the lotion is carried out under aseptic conditions.

### EXAMPLE 20

### DUSTING POWDER

| Ingredients | Amount |
|---|---|
| NaPA | 25 g. |
| Sterilized absorbable maize starch BP dusting powder | 25 g. |

### EXAMPLE 21

### SUPPOSITORY, RECTAL AND VAGINAL PHARMACEUTICAL PREPARATIONS

Suppositories, each weighing 2.5 g. and containing 100 mg. of NaPA are prepared as follows:

| Ingredients | Amount/1000 |
|---|---|
| Suppositories | |
| NaPA, micronized | 100 g. |
| Propylene glycol | 150 g. |
| Polyethylene glycol | |
| 4000, qs | 2500 g. |

NaPA is finely divided by means of an air micronizer and added to the propylene glycol and the mixture is passed through a colloid mill until uniformly dispersed. The polyethylene glycol is melted and the propylene glycol dispersion added slowly with stirring. The suspension is poured into unchilled molds at 40°C. Composition is allowed to cool and solidify and then removed from the mold and each suppository is foil wrapped.

The foregoing suppositories are inserted rectally or vaginally for treating neoplastic disease.

It is known that intracellular glutathione plays a major role in detoxification and repair of cellular injury by chemical and physical carcinogens. NaPA treatment of normal or tumor cells markedly induced the activity of intracellular glutathione approximately 2-10 fold depending on growth conditions. Nontoxic agents that can induce glutathione are highly desirable since these are likely to protect cells from damage by a variety of chemical carcinogens and ionizing radiation.

Taken together, the present invention demonstrates that NaPA has valuable potential in cancer prevention in cases such as high risk individuals, for example, heavy smokers with familial history of lung cancer, inherited disorders of oncogene abnormalities (Li-Fraumeni syndrome), individuals exposed to radiation, and patients in remission with residual disease. Furthermore, NaPA can be used in combination with other therapeutic agents, such as chemicals and radiation, to enhance tumor responses and minimize adverse effects such as cytotoxicity and carcinogenesis. The antitumor activity, lack of toxicity, and easy administration qualify NaPA as a preferred chemopreventive drug.

## Claims

1. A pharmaceutical composition for use as a medicament comprising a pharmaceutically effective amount of a compound of formula I:
R¹R²CH-COOH
wherein
R¹ is selected from H, CH₃, CH₃-O-, C₂H₅ or C₃H₇;
R² is 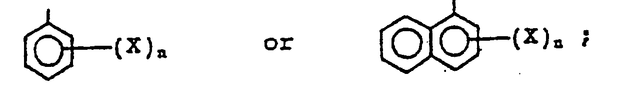
wherein X is independently selected from halogen or hydroxy, and n is 0 to 4; a stereoisomer thereof, or a pharmaceutically acceptable salt thereof, and mixtures thereof, with the proviso that if R¹ is H then R² cannot be unsubstituted phenyl or hydroxyphenyl,
together with a pharmaceutically acceptable carrier.

2. The composition of claim 1, wherein the pharmaceutically acceptable salts are selected from the group consisting of an alkali and alkaline earth metal.

3. The composition of claim 1, wherein the pharmaceutically acceptable salt is an alkali metal.

4. The composition of claim 3, wherein the alkali metal is sodium.

5. The composition of claim 1, wherein X is Cl, F, or hydroxy.

6. The composition of claim 5, wherein X is Cl.

7. The composition of claim 6, wherein R² is

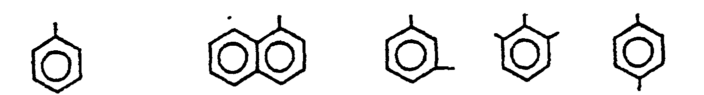

8. The composition of claim 7, wherein R' is C₃H₇.

9. The composition of claim 8, wherein R² is

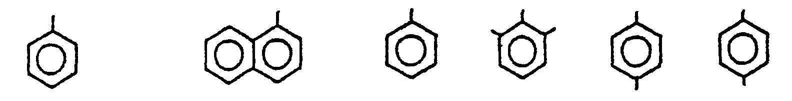

10. The composition of claim 9, wherein R² is phenyl.

11. The composition of claim 9, wherein R² is phenyl and R¹ is C₃H₇.

12. The composition of claim 9, wherein R² is naphthyl.

13. The composition of claim 9, wherein R² is m-chlorophenyl.

14. The composition of claim 1, comprising 0.010 to 99.990 weight percent of said compound.

15. The composition of claim 1, which can be administered intravenously, enterally, parenterally, intramuscularly, intranasally, subcutaneously, topically, or orally.

16. A pharmaceutical composition comprising a pharmaceutically effective amount of a compound of formula I
R¹R²CH-COOH
wherein
R¹ is selected from H, CH₃, CH₃-O-, C₂H₅ or C₃H₇;
R² is 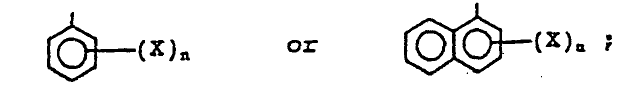
wherein X is independently selected from halogen or hydroxy and n is 0 to 4; a stereoisomer thereof, or a pharmaceutically acceptable salt thereof, and mixtures thereof as a first agent, and a second agent selected from hydroxyurea, 5-azacytidine, 5-aza-2'-deoxycytidine, suramin; retinoids; hormones; biological response modifiers selected from interferon and hematopoetic growth factors as a combined composition for use as a medicament.

17. Pharmaceutical composition according to claim 16, wherein the second agent is a compound selected from hydroxyurea, 5-aza-2'-deoxycytidine, suramin, retinoic acid and interferon.

18. Composition according to claim 16 or 17 wherein the first agent is a compound as defined in one or more of claims 2 to 13.

19. Use of a compound selected from sodium phenylbutyrate or a compound of formula I
R¹R²CH-COOH
wherein
R¹ is selected from H, CH₃, CH₃-O-, C₂H₅ or C₃H₇;
R² is 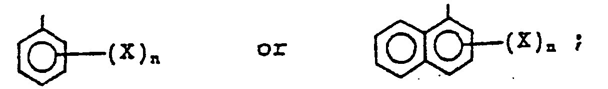
wherein X is independently selected from halogen or hydroxy and n is 0 to 4; a stereoisomer thereof, or a pharmaceutically acceptable salt thereof, and mixtures thereof, for preparing a drug for treating malignant conditions selected from prostate cancer, melanoma, glial brain tumors, AIDS-associated Kaposi's sarcoma and lymphomas, leukemia, lung adenocarcinoma, breast cancer, osteosarcoma, fibrosarcoma and squamous cancers, with the proviso that the compound is not phenylacetic acid when the condition is breast cancer.

20. The use of claim 19, wherein the compound is phenylacetic acid or the sodium salt thereof.

21. The use of claim 19, wherein phenylacetic acid or the sodium salt thereof is used concomittantly or in combination with an antitumor agent selected from the group consisting of hydroxyurea, suramin, retinoids, 5-azacytidine and 5-aza-2-deoxycytidine.

22. The use of claim 19, wherein the phenylacetic acid or the sodium salt thereof is used concomittantly or in combination with a biological response modifier selected from the group consisting of interferons, hormones and hormone-antagonists.

23. The use of claim 19, wherein the compound is sodium phenylbutyrate.

24. The use of claim 19, wherein the drug is for intravenous, enteral, parenteral, intramuscular, intranasal, subcutaneous, topical, or oral administration.

25. Use of a compound selected from sodium phenylbutyrate or a compound of formula I
R¹R²CH-COOH
wherein
R¹ is selected from H, CH₃, CH₃-O-, C₂H₅ or C₃H₇;
R² is 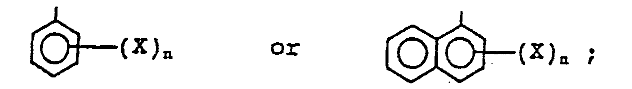
wherein X is independently selected from halogen or hydroxy and n is 0 to 4; a stereoisomer thereof, or a pharmaceutically acceptable salt thereof, and mixtures thereof as a first agent, and of a second agent selected from hydroxyurea, 5-azacytidine, 5-aza-2'-deoxycytidine, suramin; retinoids; hormones; biological response modifiers selected from interferon and hematopoetic growth factors for preparing a preparation to be used concomitantly or combined for treating cancer.

26. Use according to claim 25, wherein the second agent is selected from hydroxyurea, 5-aza-2'-deoxycitidine, suramin, retinoic acid and interferon.

## Patentansprüche

1. Pharmazeutische Zusammensetzung zur Verwendung als Arzneimittel umfassend eine pharmazeutisch wirksame Menge einer Verbindung der Formel I:
R¹R²CH-COOH
worin
R¹ ausgewählt ist aus H, CH₃, CH₃-O-, C₂H₅ oder C₃H₇;
R² 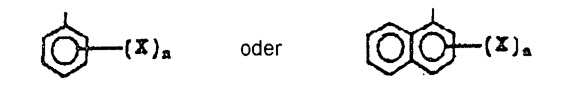
ist, worin
X unabhängig ausgewählt ist aus Halogen oder Hydroxyresten und n 0 bis 4 ist, ein Stereoisomer davon oder ein pharmazeutisch annehmbares Salz davon und Mischungen davon mit dem Vorbehalt, dass dann, wenn R¹ H ist, dass dann R² kein unsubstituierter Phenyl- oder Hydroxyphenylrest sein kann,
zusammen mit einem pharmazeutisch annehmbaren Träger.

2. Zusammensetzung nach Anspruch 1, wobei die pharmazeutisch annehmbaren Salze ausgewählt sind aus der Gruppe bestehend aus einem Alkali- und einem Erdalkalimetall.

3. Zusammensetzung nach Anspruch 1, wobei das pharmazeutisch annehmbare Salz ein Alkalisalz ist.

4. Zusammensetzung nach Anspruch 3, wobei das Alkalimetall Natrium ist.

5. Zusammensetzung nach Anspruch 1, wobei X Cl, F oder ein Hydroxyrest ist.

6. Zusammensetzung nach Anspruch 5, wobei X Cl ist.

7. Zusammensetzung nach Anspruch 6, wobei R² 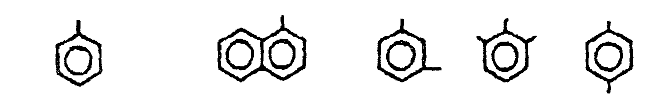 ist.

8. Zusammensetzung nach Anspruch 7, wobei R' C₃H₇ ist.

9. Zusammensetzung nach Anspruch 8, wobei R² 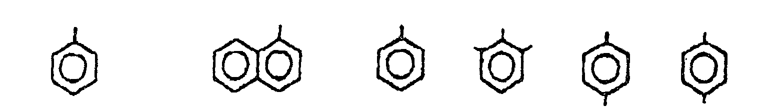 ist.

10. Zusammensetzung nach Anspruch 9, wobei R² ein Phenylrest ist.

11. Zusammensetzung nach Anspruch 9, wobei R² ein Phenylrest ist und R¹ C₃H₇ ist.

12. Zusammensetzung nach Anspruch 9, wobei R² ein Naphthylrest ist.

13. Zusammensetzung nach Anspruch 9, wobei R² ein m-Chlorphenylrest ist.

14. Zusammensetzung nach Anspruch 1, umfassend 0,010 bis 99,990 Gew.-% der Verbindung.

15. Zusammensetzung nach Anspruch 1, die intravenös, enteral, parenteral, intramuskulär, intranasal, subcutan, topisch oder oral verabreicht werden kann.

16. Pharmazeutische Zusammensetzung umfassend eine pharmazeutisch wirksame Menge einer Verbindung der Formel I:
R¹R²CH-COOH
worin
R¹ ausgewählt ist aus H, CH₃, CH₃-O-, C₂H₅ oder C₃H₇;
R² 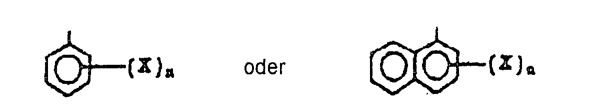 ist;
worin X unabhängig ausgewählt ist aus Halogen oder Hydroxyresten und n 0 bis 4 ist; einem Stereoisomer davon oder einem pharmazeutisch annehmbaren Salz davon und Mischungen davon als erstem Mittel, und ein zweites Mittel ausgewählt aus Hydroxyharnstoff, 5-Azacytidin, 5-Aza-2'-desoxycytidin, Suramin; Retinoiden, Hormonen; Modifikatoren des biologischen Ansprechvermögens ausgewählt aus Interferon und hämopoetischen Wachstumsfaktoren als kombinierte Zusammensetzung zur Verwendung als Arzneimittel.

17. Pharmazeutische Zusammensetzung nach Anspruch 16, wobei das zweite Mittel eine Verbindung ist ausgewählt aus Hydroxyharnstoff, 5-Aza-2'-desoxycytidin, Suramin, Retinsäure und Interferon.

18. Zusammensetzung nach Anspruch 16 oder Anspruch 17, wobei das erste Mittel eine Verbindung wie in einem oder mehreren der Ansprüche 2 bis 13 definiert ist.

19. Verwendung einer Verbindung ausgewählt aus Natriumphenylbutyrat oder einer Verbindung der Formel I:
R¹R²-CH-COOH,
worin
R¹ ausgewählt ist aus H, CH₃, CH₃-O-, C₂H₅ oder C₃H₇;
R² 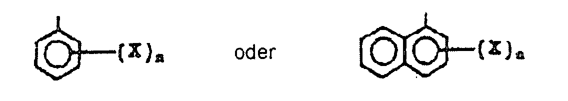
ist, worin
X unabhängig ausgewählt ist aus Halogen oder Hydroxyresten und n 0 bis 4 ist; einem Stereoisomer davon oder einem pharmazeutisch annehmbaren Salz davon und Mischungen davon zur Herstellung eines Arzneimittels zur Behandlung von malignen Zuständen ausgewählt aus Prostatakrebs, Melanomen, Glia-Hirn-Tumoren, mit AIDS in Beziehung stehenden Kaposi-Sarcoma und Lymphoma, Leukämie, Lungenadenokarzinom, Brustkrebs, Osteosarcoma, Fibrosarcoma und Schuppenzellkrebs, mit dem Vorbehalt, dass die Verbindung nicht Phenylessigsäure ist, wenn der Zustand Brustkrebs ist.

20. Verwendung nach Anspruch 19, wobei die Verbindung Phenylessigsäure oder ein Natriumsalz davon ist.

21. Verwendung nach Anspruch 19, wobei Phenylessigsäure oder das Natriumsalz zusammen oder in Kombination mit einem Antitumormittel ausgewählt aus der Gruppe bestehend aus Hydroxyharnstoff, Suramin, Retinoiden, 5-Azacytidin und 5-Aza-2-desoxycytidin verwendet wird.

22. Verwendung nach Anspruch 19, wobei die Phenylessigsäure oder das Natriumsalz davon gleichzeitig oder in Kombination mit einem Modifikator des biologischen Ansprechvermögens ausgewählt aus der Gruppe bestehend aus Interferonen, Hormonen und Hormonantagonisten verwendet wird.

23. Verwendung nach Anspruch 19, wobei die Verbindung Natriumphenylbutyrat ist.

24. Verwendung nach Anspruch 19, wobei das Arzneimittel für die intravenöse, enterale, parenterale, intramuskuläre, intranasale, subcutane, topische oder orale Verabreichung vorgesehen ist.

25. Verwendung einer Verbindung ausgewählt aus Natriumphenylbutyrat oder einer Verbindung der Formel I:
R¹R²CH-COOH
worin
R¹ ausgewählt ist aus H, CH₃, CH₃-O-, C₂H₅ oder C₃H₇;
R² 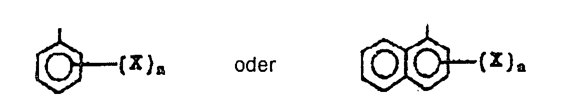 ist;
worin X unabhängig ausgewählt ist aus Halogen oder Hydroxyresten und n 0 bis 4 ist; einem Stereoisomer davon oder einem pharmazeutisch annehmbaren Salz davon und Mischungen davon als erstem Mittel, und einem zweiten Mittel ausgewählt aus Hydroxyharnstoff, 5-Azacytidin, 5-Aza-2'-desoxycytidin, Suramin; Retinoiden, Hormonen; Modifikatoren des biologischen Ansprechvermögens ausgewählt aus Interferon und hämopoetischen Wachstumsfaktoren zur Herstellung eines Präparats, das gleichzeitig oder kombiniert verwendet wird zur Behandlung von Krebs.

26. Verwendung nach Anspruch 25, wobei das zweite Mittel ausgewählt ist aus Hydroxyharnstoff, 5-Aza-2'-desoxycytidin, Suramin, Retinsäure und Interferon.

## Revendications

1. Composition pharmaceutique pour utilisation comme médicament comportant une teneur pharmaceutiquement efficace d'un composé de formule I:
R¹R²CH-COOH
dans laquelle
R¹ est choisi parmi le groupe constitué par H, CH₃, CH₃-O-, C₂H₅ ou C₃H₇,
R² est 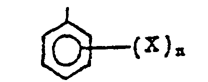 ou 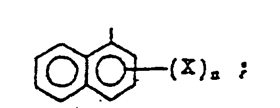
dans laquelle X est choisi indépendamment parmi un halogène ou un hydroxy, et n est compris entre 0 et 4 ; d'un stéréo-isomère de celui-ci, ou d'un sel pharmaceutiquement acceptable de celui-ci, et des mélanges de ceux-ci, à la condition que si R¹ est H alors R² ne peut pas être un phényl non substitué ou un hydroxyphényl,
ensemble avec un véhicule pharmaceutiquement acceptable.

2. Composition selon la revendication 1, dans laquelle les sels pharmaceutiquement acceptables sont choisis parmi le groupe constitué par un alcali et un métal alcalino-terreux.

3. Composition selon la revendication 1, dans laquelle le sel pharmaceutiquement acceptable est un métal alcalin.

4. Composition selon la revendication 3, dans laquelle le métal alcalin est le sodium.

5. Composition selon la revendication 1, dans laquelle X est Cl, F ou un hydroxy.

6. Composition selon la revendication 5, dans laquelle X est Cl.

7. Composition selon la revendication 6, dans laquelle R² est

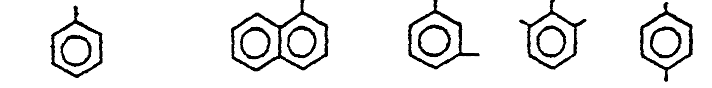

8. Composition selon la revendication 7, dans laquelle R¹ est C³H⁷.

9. Composition selon la revendication 8, dans laquelle R² est

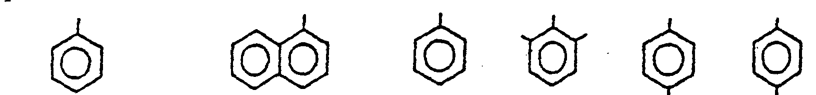

10. Composition selon la revendication 9, dans laquelle R² est le phényl.

11. Composition selon la revendication 9, dans laquelle R² est le phényl et R¹ est C₃H₇.

12. Composition selon la revendication 9, dans laquelle R² est le naphtyl.

13. Composition selon la revendication 9, dans laquelle R² est le m-chlorophényl.

14. Composition selon la revendication 1, comprenant 0,010 à 99,990 pour cent en poids du dit composé.

15. Composition selon la revendication 1, qui peut être administrée par voie intraveineuse, entérale, parentérale, intramusculaire, intranasale, sous-cutanée, topiquement ou par voie orale.

16. Composition pharmaceutique comprenant une teneur pharmaceutiquement efficace d'un composé de formule I
R¹R²CH-COOH
dans laquelle
R¹ est choisi parmi le groupe constitué par H, CH₃, CH₃-O-, C₂H₅ ou C₃H₇,
R² est 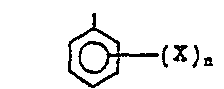 ou 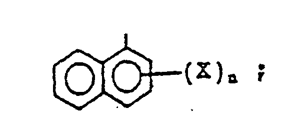
dans laquelle X est choisi indépendamment parmi un halogène ou un hydroxy, et n est compris entre 0 et 4 ; d'un stéréo-isomère de celui-ci, ou d'un sel pharmaceutiquement acceptable de celui-ci, et des mélanges de ceux-ci comme premier agent, et un second agent choisi parmi le groupe constitué par l'hydroxyurée, la 5-azacytidine, la 5-aza-2'-désoxycytidine, la suramine; des rétinoïdes; des hormones; des modificateurs de la réponse biologique choisis parmi l'interféron et les facteurs de croissance hématopoïétiques en tant que composition combinée pour une utilisation comme médicament.

17. Composition pharmaceutique selon la revendication 16, dans laquelle le second agent est un composé choisi parmi le groupe constitué par l'hydroxyurée, la 5-aza-2'-désoxycytidine, la suramine, l'acide rétinoïque et l'interféron.

18. Composition selon la revendication 16 ou 17, dans laquelle le premier agent est un composé tel que celui défini dans une ou plusieurs des revendications 2 à 13.

19. Utilisation d'un composé choisi parmi le phénylbutyrate de sodium ou un composé de formule I
R¹R²CH-COOH
dans laquelle
R¹ est choisi parmi le groupe constitué par H, CH₃, CH₃-O-, C₂H₅ ou C₃H₇,
R² est 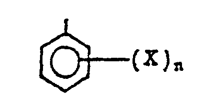 ou 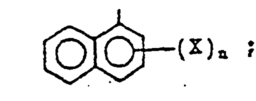
dans laquelle X est choisi indépendamment parmi un halogène ou un hydroxy, et n est compris entre 0 et 4 ; d'un stéréo-isomère de celui-ci, ou d'un sel pharmaceutiquement acceptable de celui-ci, et des mélanges de ceux-ci, pour la préparation d'un médicament pour traiter les états de malignité choisis parmi le groupe constitué par le cancer de la prostate, le mélanome, les tumeurs gliales du cerveau, les sarcomes de Kaposi et les lymphomes associés au SIDA, la leucémie, l'adénocarcinome du poumon, le fibrosarcome et les cancers squameux, à la condition que le composé ne soit pas l'acide phénylacétique dans le cas du cancer du sein.

20. Utilisation selon la revendication 19, dans laquelle le composé est l'acide phénylacétique ou son sel de sodium.

21. Utilisation selon la revendication 19, dans laquelle l'acide phénylacétique est utilisé concomitamment ou en combinaison avec un agent antitumeur choisi parmi le groupe constitué par l'hydroxyurée, la suramine, des rétinoïdes, la 5-azacytidine et la 5-aza-2-désoxycytidine.

22. Utilisation selon la revendication 19, dans laquelle l'acide phénylacétique ou son sel de sodium est utilisé concomitamment ou en combinaison avec un modificateur de la réponse biologique choisi parmi le groupe constitué par des interférons, des hormones et des antagonistes des hormones.

23. Utilisation selon la revendication 19, dans laquelle le composé est le phénylbutyrate de sodium.

24. Utilisation selon la revendication 19, dans laquelle le médicament est à administration intraveineuse, entérale, parentérale, intramusculaire, intranasale, sous-cutanée, topique ou orale.

25. Utilisation d'un composé choisi parmi le phénylbutyrate de sodium ou un composé de formule I
R¹R²CH-COOH
dans laquelle
R¹ est choisi parmi H, CH₃, CH₃-O, C₂H₅ ou C₃H₇;
R² est 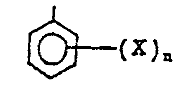 ou 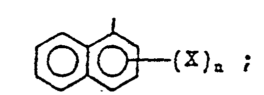
dans laquelle X est choisi indépendamment parmi un halogène ou un hydroxy, et n est compris entre 0 et 4 ; d'un stéréo-isomère de celui-ci, ou d'un sel pharmaceutiquement acceptable de celui-ci, et des mélanges de ceux-ci comme premier agent, et comme second agent , un agent choisi parmi l'hydroxyurée, la 5-azacytidine, la 5-aza-2'-désoxycytidine, la suramine; des rétinoïdes; des hormones; des modificateurs de la réponse biologique choisis parmi l'interféron et des facteurs de croissance hématopoïétiques pour préparer une préparation à utiliser concomitamment ou combinée pour traiter le cancer.

26. Utilisation selon la revendication 25, dans laquelle le second agent est choisi parmi l'hydroxyurée, la 5-aza-2'-désoxycitidine, la suramine, l'acide rétinoïque et l'interféron.
